# EUROPEAN PATENT APPLICATION

(11) **EP 1 938 812 A1**
(43) Date of publication of application: **02.07.2008**
(21) Application number: 06127175.5
(22) Date of filing: 22.12.2006
(51) Int. Cl.: A61K 31/165, A61K 31/506, A61P 13/12, A61P 9/08, A61P 9/12

(54) **Pharmaceutical composition using aliskiren and avosentan**

(71) Applicant: Speedel Pharma AG, 4051 Basel (CH)
(72) Inventor: The designation of the inventor has not yet been filed
(74) Representative: Maué, Paul Georg

(57) **Abstract**

The present invention relates to a pharmaceutical composition and method of achieving a therapeutic effect including, but not limited to, the treatment of hypertension, kidney or heart disease in an animal, preferably a mammal including a human subject, using (i) SPP100/aliskiren or a pharmaceutically acceptable salt thereof in combination with (ii) SPP301/avosentan or a pharmaceutically acceptable salt thereof and (iii) a pharmaceutically acceptable carrier.

## Description

The invention relates to a pharmaceutical composition and method of achieving a therapeutic effect including, but not limited to, the treatment of hypertension, kidney or heart disease in an animal, preferably a mammal including a human subject, using (i) aliskiren (SPP100) or a pharmaceutically acceptable salt thereof in combination with (ii) avosentan (SPP301) or a pharmaceutically acceptable salt thereof and (iii) a pharmaceutically acceptable carrier.

Accordingly, the invention of combined administration of the renin inhibitor SPP100/aliskiren together with the endothelin receptor antagonist SPP301/avosentan relates also to a method for the prevention of, delay of progression of and treatment of a disease or condition selected from the group consisting of: hypertension of defined or undefined etiology, such as essential, renovascular, nephrosclerotic, malignant hypertension and its renal complications in form of vascular lesions, microangiopathic hemolytic anemia, chronic and acute renal failure upon but not limited to glomerulonephritis, pyelonephritis, tubulointerstitial nephritis or nephritic syndrome, as well as its cardiac complications in form of chronic heart failure, ischemia reperfusion injury and myocardial infarct.

More specifically SPP100/aliskiren corresponds to the chemical formula 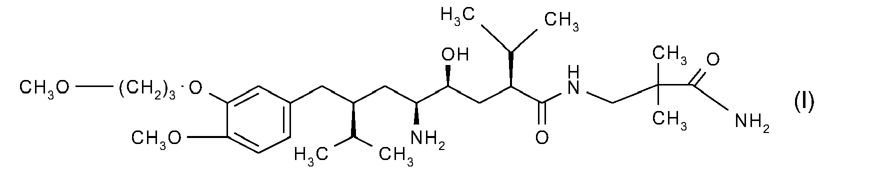 and pharmaceutically acceptable salts thereof, in particular the hemi-fumarate salt thereof. SPP100/aliskirin is known as a renin inhibitor and a method of preparation is disclosed in EP 678503.

More specifically SPP301/avosentan corresponds to the chemical formula 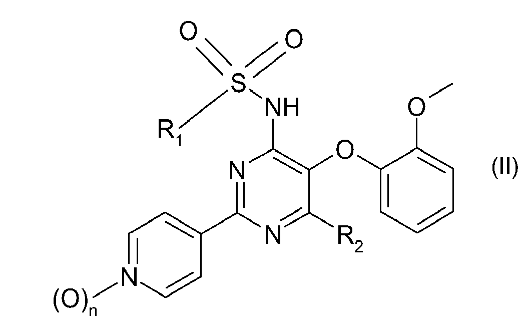 wherein
R₁ is pyridyl or thiazolyl, any of which may optionally be substituted with C₁₋₈alkyl or
C₂₋₈alkenyl; and
a) R₂ is methoxy and n is zero or one; or
b) R₂ is chlorine and n is zero
   and pharmaceutically acceptable salts thereof.

SPP301/avosentan is known as an inhibitor of endothelin receptors and a method of preparation is disclosed in WO 00/52007.

More particularly, the present invention relates to the following compounds of formula (II): R₁ is preferably, optionally substituted with d₁₋₈alkyl or C₂₋₈alkenyl, 2-pyridyl or 2-thiazolyl and most preferably, optionally substituted with C₁₋₈alkyl or C₂₋₈alkenyl, 2-pyridyl.

d₁₋₈alkyl or C₂₋₈alkenyl are branched or straight chain radicals, for example methyl, ethyl, n-propyl, isopropyl, n-butyl, isobutyl, t-butyl, vinyl, 1-propenyl, allyl, isopropenyl, 1-butenyl, 2-butenyl, 3-butenyl and the like. Preferred are said residues which have up to (and including) four carbon atoms. Most preferred is methyl.

Particularly preferred are compounds of formula (II) wherein
R₁ is 2-pyridyl optionally substituted with C₁₋₄alkyl; and
R₂ is methoxy and n is zero
and pharmaceutically acceptable salts thereof.

Most preferred SPP301/avosentan is 5-methyl-pyridine-2-sulfonic acid [6-methoxy-5-(2-methoxy-phenoxy)-2-pyridin-4-yl-pyrimidin-4-yl]-amide and pharmaceutically acceptable salts thereof.

One early manifestation of hypertension-induced kidney damage and nephropathies in humans is increased albumin excretion into the urine that may progress to nephritic-range proteinuria. The morphological substrates for these functional abnormalities of the glomerular filter apparatus include glomerular hypertrophy, thickening of the glomerular basement membrane, loss of podocytes and expansion of mesangial extracellular matrix. In addition, ultrafiltered proteins accelerate via inflammatory processes the progressive deterioration of glomerular filtration capacity. Therefore, restoring the permselective function of the kidney barrier to proteins limits the decline of glomerular filtration and ultimately prevents the loss of kidney function. Moreover, it has been suggested that reduction of urinary albumin excretion is associated with improvement of cardiovascular prognosis.

The pharmacological effect of a drug or a drug combination on the development of urinary protein excretion in the context of hypertension is used clinically as surrogate marker to assess the benefits of a drug therapy for the prevention of kidney failure.

Thus, clinical studies with angiotensin-converting enzyme inhibitors, angiotensin receptor antagonists, calcium channel blocker demonstrated the benefits of blood pressure control and reduction of albuminuria for the prevention of renal complications. Particularly, inhibitors of the renin-angiotensin-aldosterone system have been able to reduce urinary albumin excretion more than expected from the reduction of blood pressure alone (Van de Wal and Gansevoort, Expert Opinion Pharmacotherapies 2006; 7:2505-2520).

The herein described invention shows a synergistic efficacy of a two drug combination consisting of the renin inhibitor SPP100/aliskiren together with the selective endothelin receptor A antagonist SPP301/avosentan on the development of albuminuria in the context of underlying hypertension. The efficacy of this drug combination has been established in a preclinical animal model of hypertension-mediated renal complications. This model develops malignant hypertension that results in marked excretion of albumin in the urine leading ultimately to kidney failure. Therefore, this model closely reflects the clinically observed condition of hypertension with renal complications.

The combined administration of the renin inhibitor SPP100/aliskiren together with the selective endothelin receptor antagonist SPP301/avosentan in conditions characterized of hypertension with renal complications may offer following synergistic benefits:
(i) increased efficiency to protect the kidneys from systemic hypertension and resulting nephropathologies caused by glomerular pressure increases, inflammatory reactions, glomerular basement alterations or changes in podocyte and mesangial cell functions as observed by the loss of proteins, particularly albumin, into the urine
(ii) Improved safety profile as the two complementary drug classes can each be lower dosed in order to achieve a corresponding therapeutic effect. Particularly, the application of high doses of SPP100/aliskiren is not necessary to achieve a better nephroprotective effect and likewise, high doses of SPP301/avosentan are not necessary to achieve significant blood pressure reductions.

The term "combination" as used throughout the description of the instant invention is meant to include simultaneous or sequential, in any order, administration, separately or in a fixed combination.

The term "pharmaceutically acceptable salts" as used throughout the description of the instant invention is meant to encompass salts with inorganic or organic acids, such as hydrochloric acid, hydrobromic acid, nitric acid, sulfuric acid, phosphoric acid, citric acid, formic acid, maleic acid, acetic acid, succinic acid, tartaric acid, fumaric acid, methanesulfonic acid, p-toluenesulfonic acid and the like.

Pharmaceutically acceptable salts of compounds having salt-forming groups are in particular acid addition salts, salts with bases, or, in the presence of a plurality of salt-forming groups, in some cases also mixed salts or internal salts.

Pharmaceutically acceptable salts are formed, for example, from compounds of formula (I) or (II) with an acidic group, for example a carboxyl or sulfonyl group, and are, for example, the salts thereof with suitable bases such as non-toxic metal salts derived from metals of group la, Ib, IIa and IIb of the Periodic Table of the Elements, for example alkali metal, in particular lithium, sodium, or potassium, salts, alkaline earth metal salts, for example magnesium or calcium salts, and also zinc salts and ammonium salts, including those salts which are formed with organic amines, such as optionally hydroxy-substituted mono-, di- or trialkylamines, in particular mono-, di- or tri(lower alkyl)amines, or with quaternary ammonium bases, e.g. methyl-, ethyl-, diethyl- or triethylamine, mono-, bis- or tris(2-hydroxy(lower alkyl))amines, such as ethanol-, diethanol- or triethanolamine, tris(hydroxymethyl)methylamine or 2-hydroxy-tert-butylamine, N,N-di(lower alkyl)-N-(hydroxy(lower alkyl))amine, such as N,N-di-N-dimethyl-N-(2-hydroxyethyl)amine, or N-methyl-D-glucamine, or quaternary ammonium hydroxides such as tetrabutyl ammoniumhydroxide. The compounds of formula (I) or (II) having a basic group, for example an amino group, may form acid addition salts, for example with suitable inorganic acids, e.g. hydrohalic acid such as hydrochloric acid, hydrobromic acid, sulfuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulfonic or phosphonic acids or N-substituted sulfamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, and also amino acids, for example the alpha-amino acids mentioned above, and also methanesulfonic acid, ethanesulfonic acid, 2-hydroxyethanesulfonic acid, ethane-1,2-disulfonic acid, benzenesulfonic acid, 4-methylbenzenesulfonic acid, naphthalene-2-sulfonic acid, 2- or 3-phosphoglycerate, glucose 6-phosphate, N-cyclohexylsulfamic acid (with formation of the cyclamates) or with other acidic organic compounds such as ascorbic acid.

The term "synergistic" as used throughout the description of the instant invention means that the effect achieved with the methods and compositions of the present invention is greater than the sum of the effects that result from methods and compositions comprising the active ingredients of this invention separately.

The term "treatment" as used throughout the description of the instant invention is meant to include also "prevention" and "delay of progression". In particular, the term "treatment" comprises the reduction in mortality rates.

The present invention relates to the use of (i) SPP100/aliskiren or a pharmaceutically acceptable salt thereof in combination with (ii) SPP301/avosentan or a pharmaceutically acceptable salt thereof for the manufacture of a medicament, in particular a medicament for the treatment of hypertension, kidney or heart disease.

Furthermore, the present invention relates to a method of treatment of hypertension, kidney or heart disease, which comprises administering an effective hypertension, kidney or heart disease treating amount of (i) SPP100/aliskiren or a pharmaceutically acceptable salt thereof in combination with (ii) SPP301/avosentan or a pharmaceutically acceptable salt thereof to a, preferably, human being or a mammalian animal.

The pharmaceutical compositions may be administered orally, for example in the form of tablets, coated tablets, dragees, hard or soft gelatine capsules, solutions, emulsions or suspensions. Administration can also be carried out rectally, for example using suppositories; locally or percutaneously, for example using ointments, creams, gels or solutions; or parenterally, e.g. intravenously, intramuscularly, subcutaneously, intrathecally or transdermally, using for example injectable solutions. Furthermore, administration can be carried out sublingually or as opthalmological preparations or as an aerosol, for example in the form of a spray.

For the preparation of tablets, coated tablets, dragees or hard gelatine capsules the compounds of the present invention may be mixed with pharmaceutically inert, inorganic or organic excipients. Examples of suitable excipients for tablets, dragees or hard gelatine capsules include lactose, maize starch or derivatives thereof, talc or stearic acid or salts thereof.

Suitable excipients for use with soft gelatine capsules may include for example vegetable oils, waxes, fats, semi-solid or liquid polyols etc..

For the preparation of solutions and syrups, excipients which may be used include for example water, polyols, saccharose, invert sugar and glucose.

For injectable solutions, excipients which may be used include for example water, alcohols, polyols, glycerine, and vegetable oils.

For suppositories, and local or percutaneous application, excipients which may be used include for example natural or hardened oils, waxes, fats and semi-solid or liquid polyols.

The pharmaceutical compositions may also contain preserving agents, solubilising agents, stabilising agents, wetting agents, emulsifiers, sweeteners, colorants, odorants, salts for the variation of osmotic pressure, buffers, coating agents or antioxidants. As mentioned earlier, they may also contain other therapeutically valuable agents.

It is a prerequisite that all adjuvants used in the manufacture of the preparations are generally recognized as safe.

Preferred forms of use are intravenous, intramuscular or oral administration, most preferred is oral administration. The dosages in which the compounds of formula (I) and (II) are administered in effective amounts depend on the nature of the specific active ingredient, the age and the requirements of the patient and the mode of application In general, dosages of about 0.01-10 mg/kg body weight per day come into consideration.

For above compounds preference is given to commercially available compounds or those compounds which have been approved by a health authority.

A further object of the instant invention is a kit for the treatment of hypertension, kidney or heart disease comprising
a) an amount of a compound of formula (I) in a first unit dosage form;
b) an amount of a compound of formula (II) in a second unit dosage form; and
c) a container containing said first and second unit dosage forms.

In a variation thereof, the instant invention likewise relates to a "kit-of-parts", in the sense that the components to be combined according to the instant invention can be dosed independently or by use of different fixed combinations with distinguished amounts of the components, i.e. simultaneously or at different time points.

### EXAMPLES

The beneficial effects of the present invention can, for example, be demonstrated in a preclinical test model as follows:

### Method

An animal model for spontaneously malignant hypertension with renal complications is represented by double transgenic rats over-expressing both, the gene for human renin and the gene for human angiotensinogen. This double transgenic rat strain is produced by crossbreeding two transgenic strains, one for human angiotensinogen with the endogenous promoter and one for human renin with the endogenous promoter. Neither single transgenic strain is hypertensive. The double transgenic rats, both males and females, develop severe hypertension (mean systolic pressure, approximately 200 mm Hg) and die after a mean of 55 days if untreated (Luft et al., Hypertension 1999; 33:212-218). The fact that human renin can be studied in the rat is a unique feature of this model and allows the extrapolation to clinical high renin hypertension.

The combination according to the present invention comprising the renin inhibitor SPP100/aliskiren or a pharmaceutically acceptable salt thereof together with the selective endothelin receptor antagonist SPP301/avosentan or a pharmaceutically acceptable salt thereof can be administered by various routes of administration but are tested in this example orally by gavage. Each agent can be tested over a wide range of dosages to determine the optimal drug level for each agent in combination to elicit the maximal response. Each study group is best performed in which the effects of the combination treatment group are determined at the same time as the individual components are evaluated.

Although drug effects may be observed upon short term treatment, it is preferable to observe responses in a chronic setting as shown below in which experiments were done over a three to seven week observation period. The long-term duration study is of sufficient duration to allow for the full development of compensatory responses to occur and therefore, the observed effect will most likely depict the actual responses of the test system representing sustained or persistent effects.

### Statistical Analysis

The combination therapy can be compared to that of the respective monotherapies by determining the maximum change in 24 hour urinary protein content at a given day. All values are represented as the group mean ± standard error of the mean (SEM). Statistical significance is obtained when p < 0.05. The albuminuria values for each of the treatment groups can be compared statistically using a one-way ANOVA followed by the appropriate post-hoc analysis, for example by performing a Bonferoni test.

### Study design

Four week old double transgenic rats were treated daily by oral gavage with SPP100/aliskiren at a dose of 10 mg/kg, SPP301/avosentan at doses of 10 and 30 mg/kg and combinations of SPP100/aliskiren with SPP301/avosentan at the same doses. After three, five and seven weeks of drug treatment, 24 hour urine samples are collected using metabolic cages. The urinary albumin content is measured by ELISA using a commercial kit (Celltrend GmbH, Luckenwalde, Germany).

### Results

The administration of the renin inhibitor SPP100/aliskiren resulted in mean urinary albumin excretions of up to 22.3 mg after seven weeks of treatment. In comparison, with the administration of SPP301/avosentan, be at 10 or 30 mg/kg daily dosing, urinary albumin contents of to 39 mg were observed. The combination of SPP100/aliskiren with
SPP301/avosentan had a far superior anti-albuminuric effect than either drug alone. The drug combinations yielded urinary albumin contents of 3.2 mg (with a 10 mg/kg SPP301/avosentan dose) and 2.8 mg (with a 30 mg/kg SPP301/avosentan dose) after seven weeks of dosing. The combination therapy was statistically superior to each monotherapy with a probability level of p<0.5 using a one-way ANOVA and a Bonferoni test. Thus, a synergistic effect on the development of albuminuria in this animal model for malignant hypertension with renal complications can be shown by the administration of SPP100/aliskiren and SPP301/avosentan in combination in comparison to the administration of the individual drugs.

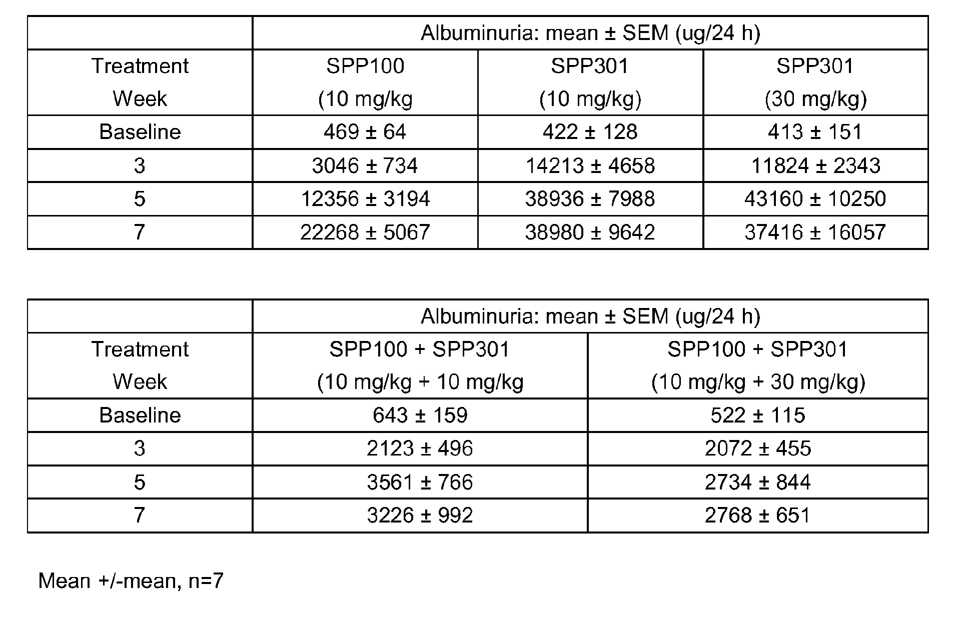

## Claims

1. Pharmaceutical composition comprising (i) SPP100/aliskiren or a pharmaceutically acceptable salt thereof, (ii) SPP301/avosentan or a pharmaceutically acceptable salt thereof and (iii) a pharmaceutically acceptable carrier.

2. Use of (i) SPP100/aliskiren or a pharmaceutically acceptable salt thereof and (ii) SPP301/avosentan or a pharmaceutically acceptable salt thereof for the preparation of a medicament for the treatment of hypertension, kidney or heart disease.

3. A method of treatment of hypertension, kidney or heart disease, which comprises administering an effective hypertension, kidney or heart disease treating amount of (i) SPP100/aliskiren or a pharmaceutically acceptable salt thereof in combination with (ii) SPP301/avosentan or a pharmaceutically acceptable salt thereof to a, preferably, human being or a mammalian animal.

4. Kit for the treatment of hypertension, kidney or heart disease comprising
a) an amount of a compound of formula (I) in a first unit dosage form;
b) an amount of a compound of formula (II) in a second unit dosage form; and
c) a container containing said first and second unit dosage forms.

5. Pharmaceutical composition, use, method or kit according to any one of claims 1 to 4 wherein SPP100/aliskiren corresponds to the chemical formula 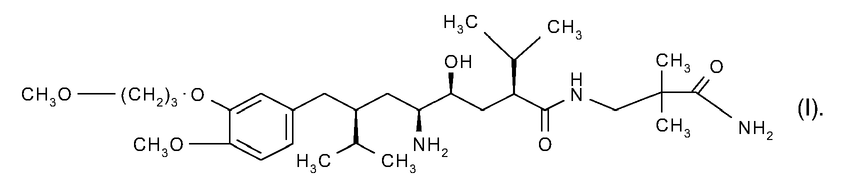 or a pharmaceutically acceptable salt thereof, in particular the hemi-fumarate salt thereof.

6. Pharmaceutical composition, use, method or kit according to any one of claims 1 to 4 wherein SPP301/avosentan corresponds to the chemical formula 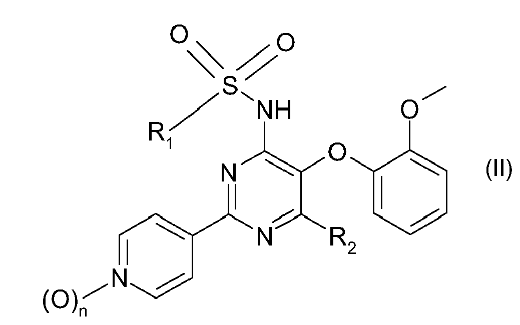 wherein
R₁ is pyridyl or thiazolyl, any of which may optionally be substituted with C₁₋₈alkyl or C₂₋₈alkenyl; and
a) R₂ is methoxy and n is zero or one; or
b) R₂ is chlorine and n is zero
or a pharmaceutically acceptable salt thereof.

7. Pharmaceutical composition, use, method or kit according to any one of claims 1 to 4 wherein SPP301/avosentan is 5-methyl-pyridine-2-sulfonic acid [6-methoxy-5-(2-methoxy-phenoxy)-2-pyridin-4-yl-pyrimidin-4-yl]-amide or a pharmaceutically acceptable salt thereof.
